# EUROPÄISCHE PATENTSCHRIFT

(11) **EP 2 269 042 B1**
(45) Veröffentlichungstag und Bekanntmachung des Hinweises auf die Patenterteilung: **24.08.2011**
(21) Anmeldenummer: 09733896.6
(22) Anmeldetag: 21.04.2009
(51) Int. Cl.: G01N 23/04, G01T 1/29

(54) **RÖNTGENCOMPUTERTOMOGRAPH UND VERFAHREN ZUR UNTERSUCHUNG EINES BAUTEILS MITTELS RÖNTGENCOMPUTERTOMOGRAPHIE**
X-RAY COMPUTER TOMOGRAPH AND METHOD FOR INVESTIGATING A COMPONENT BY MEANS OF X-RAY COMPUTER TOMOGRAPHY
TOMODENSITOMÈTRE À RAYONS X ET PROCÉDÉ DE CONTRÔLE D'UN ÉLÉMENT STRUCTURAL PAR TOMODENSITOMÉTRIE

(30) Priorität: 25.04.2008 DE 102008020948
(43) Veröffentlichungstag der Anmeldung: 05.01.2011
(73) Patentinhaber: Fraunhofer-Gesellschaft zur Förderung der angewandten Forschung e.V., 80686 München (DE)
(72) Erfinder: HANKE, Randolf, 90617 Puschendorf (DE); FUCHS, Theobald, 90429 Nürnberg (DE); SCHÖN, Tobias, 90455 Nürnberg (DE); MAISL, Michael, 66629 Freisen (DE); KASPERL, Stefan, 91058 Erlangen (DE)
(74) Vertreter: Rau, Schneck & Hübner
(86) Internationale Anmeldenummer: PCT/EP2009/002893
(87) Internationale Veröffentlichungsnummer: WO 2009/129994

(56) Entgegenhaltungen:
- WO-A-00/46592
- WO-A-03/085416
- US-A- 5 740 224
- US-A1- 2003 035 513
- US-A1- 2004 109 532
- US-B1- 6 459 756

## Beschreibung

Die Erfindung betrifft einen Röntgencomputertomographen zur Untersuchung eines Bauteils mittels Röntgencomputertomographie. Ferner betrifft die Erfindung ein Verfahren zur Untersuchung eines Bauteils mittels Röntgencomputertomographie.

Im industriellen Bereich wird die Röntgencomputertomographie (CT) zur zerstörungsfreien und berührungslosen Untersuchung von Bauteilen eingesetzt. Die Röntgencomputertomographie ermöglicht beispielsweise die Detektion von Materialfehlern sowie deren Charakterisierung hinsichtlich ihrer Art und Lage im Bauteil. Darüber hinaus besteht seitens der Industrie ein Bedarf, das Bauteil mittels der Röntgencomputertomographie geometrisch zu vermessen, so dass das Einhalten von Fertigungstoleranzen, insbesondere bei verdeckten inneren Strukturen des Bauteils, überprüft werden kann.

Bei bekannten Verfahren zur Untersuchung und geometrischen Vermessung von Bauteilen wird das zu untersuchende Bauteil mit Röntgenstrahlung in Form eines im Wesentlichen zweidimensionalen Fächerstrahls bestrahlt, wobei das Bauteil während des Bestrahlens einmal rotiert. Mittels eines Ein- oder Mehrzeilendetektors werden Fächerstrahldaten der bestrahlen im Wesentlichen zweidimensionalen Schicht des Bauteils gemessen. Die eingesetzten Mehrzeilendetektoren weisen eine wesentlich kleinere Zeilen- als Spaltenanzahl auf, beispielsweise 16 Zeilen zu 1024 Spalten. Aus den Fächerstrahldaten kann die bestrahlte zweidimensionale Schicht des Bauteils exakt rekonstruiert werden. Ein derartiges Verfahren ist beispielsweise aus der EP 0 875 751 A1 bekannt. Nachteilig bei diesem Verfahren ist, dass zur Gewinnung von dreidimensionalen Volumendaten des Bauteils ein erheblicher Zeitaufwand erforderlich ist, da das Bauteil Schicht für Schicht erfasst werden muss. Aufgrund des erforderlichen Zeitaufwands ist dieses Verfahren für eine industrielle geometrische Vermessung von Bauteilen ungeeignet.

Aus der WO 00/46592 A2 ist ein Röntgencomputertomograph zur Untersuchung von Turbinenschaufeln bekannt. Die Turbinenschaufeln werden relativ zu der Strahlungsquelle auf einer helixförmigen Bahn bewegt, wobei die Strahlungsquelle eine kegelförmige Strahlungsgeometrie und der Detektor einen entsprechenden zweidimensionalen Aufbau hat. Hierdurch kann beispielsweise die Wanddicke von innenliegenden Kanälen der Turbinenschaufeln überprüft werden.

Aus der US 5,740,224 ist ein Verfahren zur Untersuchung von Teilen mit Übergröße bekannt. Mittels einer Strahlungsquelle mit dreidimensionaler Strahlungsgeometrie werden Teil-Projektionen aufgenommen und zu einer vollständigen virtuellen Projektion des Bauteils zusammengefügt. Der Bauteilträger ist relativ zu der Strahlungsquelle und dem Detektor in allen drei Raumrichtungen linear verfahrbar.

Aus der US 6,459,756 ist ein Röntgencomputertomograph bekannt, bei dem das Bauteil relativ zu der Strahlungsquelle und dem Detektor auf einer helixförmigen Bahn bewegt wird. Der Pitch der helixförmigen Bahn ist einstellbar.

Aus der US 2004/0109532 A1 ist ein Röntgencomputertomograph zur Untersuchung von Containern bekannt. Der Bauteilträger ist um eine vertikale Achse drehbar und entlang dieser Achse linear verfahrbar.

Aus der US 2003/0035513 A1 ist ein medizinischer Röntgencomputertomograph bekannt, mittels dem eine Strahlenbelastung von bestimmten Bereichen, wie beispielsweise einem Fötus, vermieden werden kann. Eine daraus resultierende Neigung der Projektionsebene führt bei der Rekonstruktion zu Artefakten. Diese Artefakte werden durch eine Neigungskorrektur der Messdaten vermieden.

Aus der WO 03/085416 A2 ist ein Röntgencomputertomograph zur Untersuchung von Automobilfelgen.bekannt.

Der Erfindung liegt daher die Aufgabe zugrunde, einen Röntgencomputertomographen zu schaffen, der eine schnelle und genaue geometrische Vermessung eines Bauteils ermöglicht.

Diese Aufgabe wird erfindungsgemäß durch einen Röntgencomputertomographen mit den Merkmalen des Anspruchs 1 gelöst. Erfindungsgemäß wurde erkannt, dass Volumendaten eines zu untersuchenden Bauteils mittels Röntgenstrahlung mit einer dreidimensionalen Strahlungsgeometrie schnell gewonnen und zu einem genauen dreidimensionalen Röntgenbild des Bauteils rekonstruiert werden können, wenn die Strahlungsquellen-Detektor-Einheit und der Bauteilträger relativ zueinander eine Bewegung in mindestens zwei Bewegungsfreiheitsgraden ausführen, so dass mindestens eine einen dreidimensionalen Raum aufspannende Trajektorie erzeugt wird. Als Trajektorie wird die Bahn bezeichnet, die ein Brennfleck der Strahlungsquelle relativ zu dem zu untersuchenden Bauteil beim Bestrahlen mit Röntgenstrahlung ausführt. Aufgrund der zwei Bewegungsfreiheitsgrade können beispielsweise eine helixförmige Trajektorie (Wendelbahn oder Helixbahn) oder/und zwei kreisförmige und zueinander, insbesondere senkrecht, geneigte Trajektorien erzeugt werden. Dadurch, dass die Strahlungsquelle Röntgenstrahlung mit einer dreidimensionalen Strahlungsgeometrie erzeugt, die mittels eines als Flächendetektor ausgebildeten Mehrzeilendetektors detektiert wird, können schnell Volumendaten des Bauteils gewonnen werden, ohne dass das Bauteil Schicht für Schicht erfasst werden muss. Als Flächendetektoren werden Detektoren mit einem Verhältnis von Detektorhöhe zu Detektorbreite von mindestens 1/8, insbesondere von mindestens 1/4, und insbesondere von 1/2 verstanden, beispielsweise ein Mehrzeilendetektor mit 256 Zeilen und 1024 Spalten. Die Begriffe Detektorhöhe und Detektorbreite sind so zu verstehen, dass die Detektorhöhe maximal so groß wie die Detektorbreite sein kann. Die derartig gewonnenen Volumendaten können mittels der Rekonstruktionseinheit zu einem genauen dreidimensionalen Röntgenbild rekonstruiert werden. Insbesondere werden die mittels des Flächendetektors gemessenen Volumendaten mittels der Rekonstruktionseinheit unmittelbar in die Volumeneinheiten (Voxel) des dreidimensionalen Röntgenbildes umgerechnet, wodurch eine Beeinträchtigung der Bildqualität aufgrund von Zwischenschritten, wie beispielsweise der Rekonstruktion und dem Zusammenfügen von einzelnen Schichten des Bauteils, vermieden werden kann. Die Bildqualität ist über das gesamte Bauteil homogen und isotrop. Eine Beeinträchtigung der Bildqualität mit ansteigendem Kegelwinkel tritt nicht auf. Dadurch, dass die Bildqualität über das gesamte Bauteil homogen und isotrop ist, können mittels der Geometrieerfassungseinheit dimensionelle Messdaten ortsunabhängig mit einer hohen Genauigkeit bestimmt werden. Beispielsweise kann das Einhalten von Fertigungstoleranzen des Bauteils überprüft werden. Der erfindungsgemäße Röntgencomputertomograph erlaubt somit ein schnelles und genaues geometrisches Vermessen, insbesondere von innenliegenden und verdeckten Strukturen, des Bauteils.

Ein Verlagern des Bauteilträgers entlang der Längsachse zwischen der Strahlungsquelle und dem Flächendetektor ermöglicht das Erzeugen von Projektionen mit einer gewünschten Vergrößerung. Je näher das zu untersuchende Bauteil an der Strahlungsquelle angeordnet ist, desto größer ist die Projektion des Bauteils auf dem Flächendetektor. Vorteilhafterweise ist eine Projektion des Bauteils mit einer Vergrößerung von mehr als 2, insbesondere von mehr als 10, und insbesondere von mehr als 50, erzeugbar. Ein Verschwenken des Flächendetektors um eine quer zu diesem verlaufende Schwenkachse ermöglicht das Ausrichten des Flächendetektors zu der mindestens einen Trajektorie. Bei einer helixförmigen Trajektorie kann der Flächendetektor entsprechend dem Pitch, das heißt der Steilheit der helixförmigen Trajektorie, ausgerichtet werden, so dass die Zeilen- bzw. Spaltenrichtung entlang einer Tangente der helixförmigen Trajektorie orientiert ist. Durch das Ausrichten wird einerseits der Flächendetektor optimal ausgenutzt und andererseits die Rekonstruktion der detektieren Röntgenstrahlung zu einem dreidimensionalen Röntgenbild vereinfacht. Insbesondere ist bei einer entsprechenden Ausrichtung des Flächendetektors eine Interpolation der detektierten Röntgenstrahlung - wie dies ohne eine Ausrichtung erforderlich wäre - zwischen den einzelnen Pixeln des Flächendetektors nicht erforderlich, wodurch eine hohe Auflösung erzielt wird. Weiterhin kann die Filteroperation, die bei der Rekonstruktion unverzichtbar ist und in vorbestimmter Richtung bezüglich des Flächendetektors durchzuführen ist, aufgrund der Ausrichtung direkt auf die primär gemessenen Daten - ohne vorherige Sortierung und/oder Interpolation - angewandt werden. Hierdurch wird ebenfalls eine hohe Bildqualität erzielt. Dies gilt entsprechend bei mehreren zueinander geneigten Trajektorien.

Eine Geometrieerfassungseinheit nach Anspruch 2 ermöglicht ein einfaches Bestimmen von dimensionellen Messdaten des Bauteils. Die Bauteiloberfläche wird beispielsweise derart bestimmt, dass jeder Volumeneinheit (Voxel) des dreidimensionalen Röntgenbildes ein Dichtewert zugeordnet wird, wobei sich die Bauteiloberfläche als Fläche gleicher Dichtewerte ergibt. Die Bauteiloberfläche als Übergang zwischen Material und Luft kann auf diese Weise einfach gefunden werden. Insbesondere die innenliegende Bauteiloberfläche, die zur Vermessung in herkömmlicher Art nicht zugänglich ist, kann mittels der Geometrieerfassungseinheit ermittelt werden.

Eine Geometrieerfassungseinheit nach Anspruch 3 optimiert den Speicherund Rechenaufwand beim Ermitteln der Bauteiloberfläche. Durch verbundene Dreiecksflächen kann die Bauteiloberfläche ausreichend genau und mit geringem Speicher- und Rechenaufwand ermittelt werden.

Ein Bauteilträger nach Anspruch 4 ermöglicht eine Drehbewegung des zu untersuchenden Bauteils.

Ein Bauteilträger nach Anspruch 5 ermöglicht eine translatorische Bewegung des zu untersuchenden Bauteils. Insbesondere kann eine helixförmige Trajektorie durch eine gleichzeitige Drehbewegung und translatorische Bewegung des zu untersuchenden Bauteils erzeugt werden.

Ein Bauteilträger nach Anspruch 6 ermöglicht das Rekonstruieren von dreidimensionalen Röntgenbildern mit einer hohen Auflösung und somit ein äußerst genaues Bestimmen von dimensionellen Messdaten des Bauteils. Das Verhältnis von Vorschub pro Umdrehung zu Strahlaufweitung entlang der Drehachse wird als Pitch, insbesondere als relativer Pitch, bezeichnet und ist ein Maß für die Steilheit der helixförmigen Trajektorie. Je kleiner der Pitch ist, desto genauer ist die Auflösung des dreidimensionalen Röntgenbildes. Unter Strahlaufweitung wird die Aufweitung der detektierbaren Röntgenstrahlung entlang der Drehachse verstanden.

Eine Rekonstruktionseinheit nach Anspruch 7 stellt die Rekonstruktion des dreidimensionalen Röntgenbildes sicher, wenn die Volumendaten durch Bewegung entlang einer helixförmigen Trajektorie gewonnen werden.

Ein Bauteilträger nach Anspruch 8 ermöglicht das Erzeugen von mehreren zueinander geneigten Trajektorien.

Eine Rekonstruktionseinheit nach Anspruch 9 stellt die Rekonstruktion des dreidimensionalen Röntgenbildes sicher, wenn die Volumendaten durch Bewegung entlang zweier kreisförmiger und zueinander geneigter Trajektorien gewonnen werden.

Eine Strahlungsquellen-Detektor-Einheit nach Anspruch 10 ermöglicht die Untersuchung von Bauteilen unterschiedlicher Größe.

Eine Strahlungsquelle nach Anspruch 11 ermöglicht ein schnelles Gewinnen von Volumendaten des Bauteils. Vorteilhafterweise ist die Strahlungsquelle derart ausgebildet, dass der Kegelöffnungswinkel einstellbar ist.

Eine Strahlungsquelle nach Anspruch 12 ermöglicht das Durchstrahlen einer Vielzahl unterschiedlicher Bauteile. Mit steigender Beschleunigungsspannung können größere Volumina der Bauteile untersucht werden. Insbesondere können mit steigender Beschleunigungsspannung auch metallische Bauteile untersucht werden. Als Strahlungsquelle können je nach gewünschter Beschleunigungsspannung Röntgenröhren oder Linearbeschleuniger eingesetzt werden. Linearbeschleuniger werden insbesondere bei Beschleunigungsspannungen von mehr als 900 kV eingesetzt.

Eine Rekonstruktionseinheit nach Anspruch 13 gewährleistet eine schnelle und genaue Rekonstruktion des dreidimensionalen Röntgenbildes. Jede Volumeneinheit (Voxel) des dreidimensionalen Röntgenbildes wird unmittelbar aus gemessenen Volumendaten zu solchen Röntgenstrahlen berechnet, die an der der Volumeneinheit entsprechenden Stelle durch das Bauteil bzw. durch die Bauteilumgebung verliefen. Hierdurch wird sichergestellt, dass bei der Rekonstruktion des Röntgenbildes keine Zwischenschritte, wie beispielsweise das Rekonstruieren und Zusammenfügen von im Wesentlichen zweidimensionalen Schichten, und/oder die Interpolation bzw. Approximation von Volumeneinheiten aus Messdaten zu Röntgenstrahlen, die an zu der Volumeneinheit benachbarten Stellen durch das Bauteil bzw. die Bauteilumgebung verliefen, erforderlich ist. Somit wird bei der Rekonstruktion ein Verlust an Genauigkeit vermieden und eine hohe Bildqualität erzielt. Dies gilt insbesondere auch für Kegelöffnungswinkel gemäß Anspruch 11.

Der Erfindung liegt ferner die Aufgabe zugrunde, ein Verfahren zur Untersuchung eines Bauteils mittels Röntgencomputertomographie zu schaffen, das eine schnelle und genaue geometrische Vermessung eines Bauteils ermöglicht.

Diese Aufgabe wird erfindungsgemäß durch ein Verfahren mit den Merkmalen des Anspruchs 14 gelöst. Die Vorteile des erfindungsgemäßen Verfahrens entsprechen den bereits beschriebenen Vorteilen des erfindungsgemäßen Röntgencomputertomographen.

Weitere Merkmale und Vorteile der Erfindung ergeben sich aus der Beschreibung mehrerer Ausführungsbeispiele anhand der Zeichnung. Es zeigen:
- Fig. 1: eine perspektivische Schemadarstellung eines Röntgencom- putertomographen gemäß einem ersten Ausführungsbeispiel,

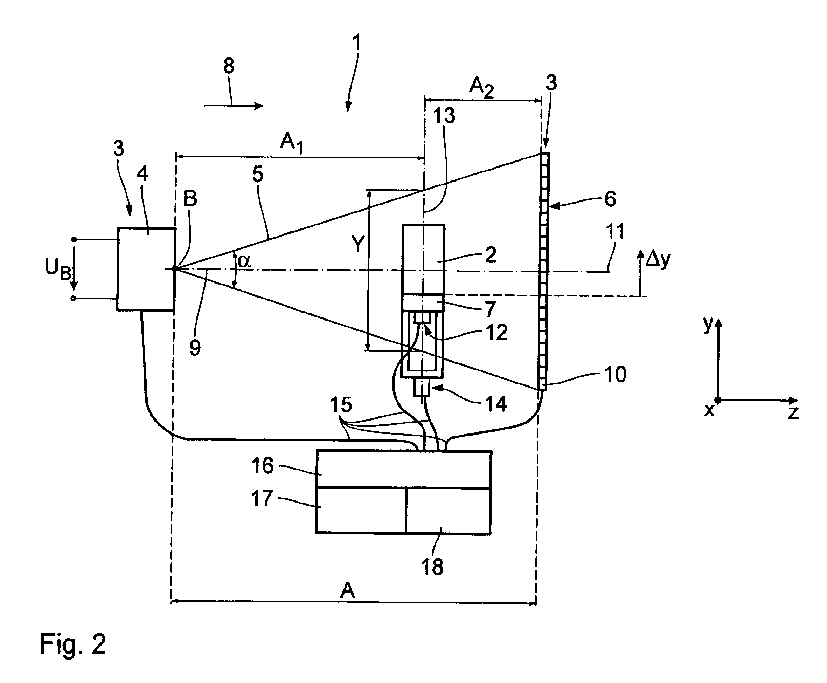

- Fig. 2: eine Seitenansicht des Röntgencomputertomographen in Fig. 1,
- Fig. 3: einen vergrößerten Ausschnitt des Röntgencomputerto- mographen in Fig. 2, und
- Fig. 4: eine Seitenansicht eines Röntgencomputertomographen ge- mäß einem zweiten Ausführungsbeispiel.

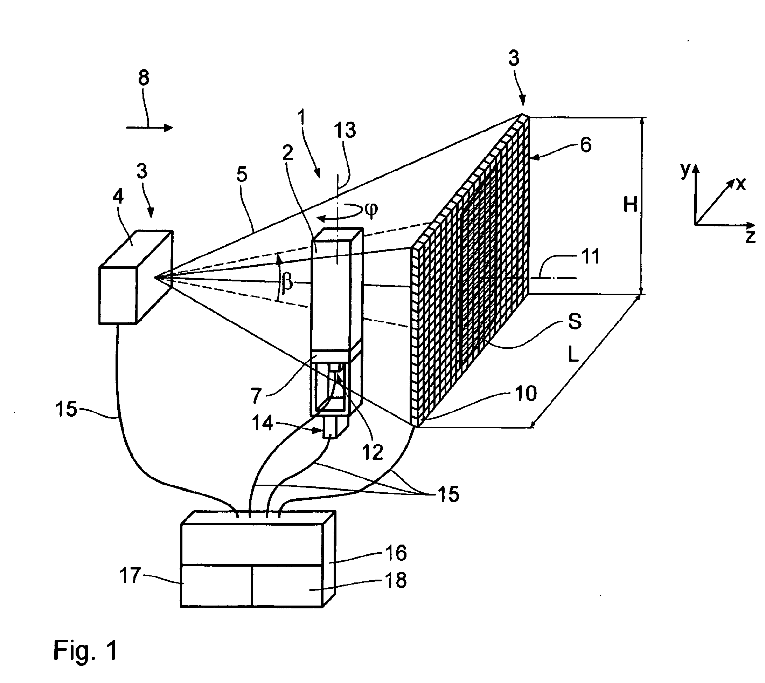

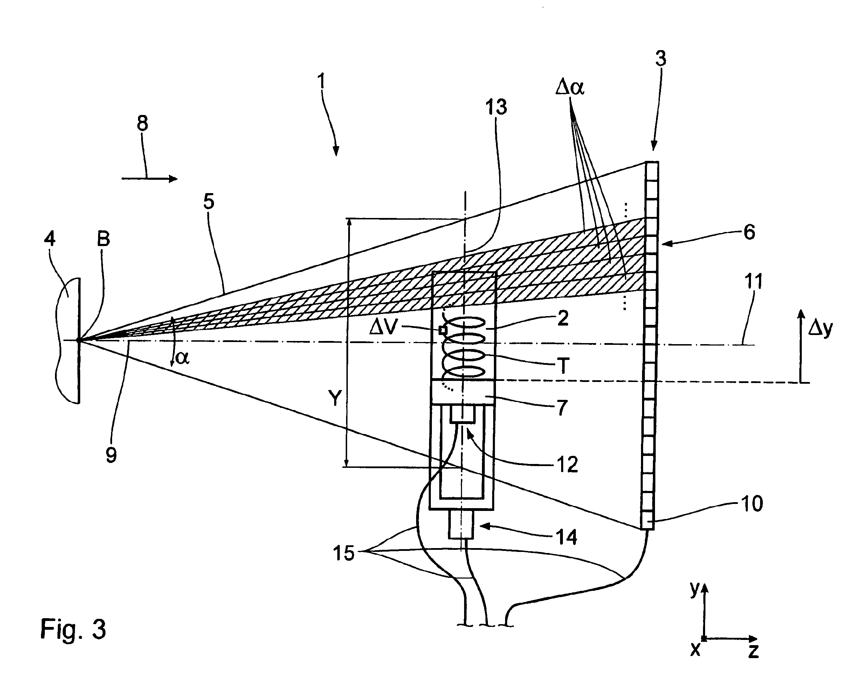

Nachfolgend wird unter Bezugnahme auf die Fig. 1 und 3 ein erstes Ausführungsbeispiel der Erfindung beschrieben. Ein Röntgencomputertomograph 1 weist zur Untersuchung eines Bauteils 2 eine Strahlungsquellen-Detektor-Einheit 3 auf. Die Strahlungsquellen-Detektor-Einheit 3 umfasst eine Strahlungsquelle 4 zum Erzeugen von Röntgenstrahlung 5 und einen Flächendetektor 6 zum Detektieren der Röntgenstrahlung 5. Zwischen der Strahlungsquelle 4 und dem Flächendetektor 6 ist ein Bauteilträger 7 angeordnet, auf dem das zu untersuchende Bauteil 2 positionierbar ist.

Mittels der Strahlungsquelle 4 ist die Röntgenstrahlung 5 mit einer dreidimensionalen Strahlungsgeometrie, insbesondere mit einer kegelförmigen Strahlungsgeometrie, erzeugbar. Die Strahlungsquelle 4 weist zum Bestrahlen des Bauteils 2 einen Kegelöffnungswinkel α von mehr als 10 °, insbesondere von mehr als 30 °, und insbesondere von mehr als 50 °, auf. Der Kegelöffnungswinkel α ist in einer y-z-Ebene definiert, die durch eine y-Richtung und eine senkrecht dazu verlaufende z-Richtung gebildet ist. Der Kegelöffnungswinkel α der Strahlungsquelle 4 kann einstellbar sein. Darüber hinaus weist die Strahlungsquelle 4 einen Fächerstrahlwinkel β auf, der in einer x-z-Ebene definiert ist. Die x-z-Ebene wird durch eine senkrecht zu der y-Richtung und der z-Richtung verlaufenden x-Richtung und der z-Richtung gebildet. Der Fächerstrahlwinkel β beträgt beispielsweise 52 °. Die Strahlungsquelle 4 ist derart ausgebildet, dass die Röntgenstrahlung 5 in einer Strahlrichtung 8 emittierbar ist, wobei die Strahlrichtung 8 im Wesentlichen entlang einer Längsachse 9 des Röntgentomographen 1 verläuft. Die Strahlungsquelle 4 weist eine Beschleunigungsspannung U_{B} auf, die mindestens 150 kV, insbesondere mindestens 450 kV, und insbesondere mindestens 900 kV, beträgt. Die Strahlungsquelle 4 ist in Abhängigkeit von der Beschleunigungsspannung U_{B} als Röntgenröhre oder als Linearbeschleuniger (LINAC) ausgebildet, wobei bei Beschleunigungsspannungen U_{B} von mehr als 900 kV Linearbeschleuniger eingesetzt werden. Der Aufbau einer Röntgenröhre und eines Linearbeschleunigers ist prinzipiell bekannt.

Der Flächendetektor 6 erstreckt sich im Wesentlichen in einer x-y-Ebene, die durch die x-Richtung und die y-Richtung gebildet ist. Die Längsachse 9 verläuft parallel zu der z-Richtung und senkrecht zu der x-y-Ebene. Der Flächendetektor 6 ist als Mehrzeilendetektor ausgebildet und weist in x-und y-Richtung eine Vielzahl von Pixeln 10 auf. Die Pixel 10 definieren in der x-Richtung Spalten und in der y-Richtung Zeilen. Aus der Zeilenanzahl und der Größe der Pixel 10 ergibt sich in y-Richtung eine Detektorhöhe H. Entsprechend ergibt sich aus der Spaltenanzahl und der Größe der Pixel 10 in x-Richtung eine Detektorbreite L. Der Flächendetektor 6 weist ein Verhältnis von Detektorhöhe H zu Detektorbreite L von mindestens 1/8, insbesondere von mindestens 1/4, und insbesondere von 1/2, auf. Beispielsweise weist der Flächendetektor 6 256 Zeilen und 1024 Spalten auf, so dass sich ein Verhältnis von Detektorhöhe H zu Detektorbreite L von 1/4 ergibt. Der Flächendetektor 6 ist um eine Schwenkachse 11 verschwenkbar. Die Schwenkachse 11 verläuft senkrecht und mittig zu dem Flächendetektor 6, so dass die Schwenkachse 11 mit der Längsachse 9 zusammenfällt. Die Strahlungsquelle 4 und der Flächendetektor 6 definieren in der z-Richtung einen Strahlungsquellen-Detektor-Abstand A. Die Strahlungsquellen-Detektor-Einheit 3 ist derart ausgebildet, dass der Strahlungsquellen-Detektor-Abstand A veränderbar ist. Hierzu ist entweder die Strahlungsquelle 4 oder der Flächendetektor 6 oder beides entlang der Längsachse 9 verlagerbar.

Der Bauteilträger 7 ist zum Drehen des zu untersuchenden Bauteils 2 mittels einer ersten Antriebseinrichtung 12 um eine Drehachse 13 drehantreibbar. Die Drehachse 13 des Bauteilträgers 7 verläuft im Wesentlichen parallel zu der y-Richtung. Die Drehachse 13 stellt einen ersten Bewegungsfreiheitsgrad F₁ bereit. Weiterhin ist der Bauteilträger 7 mittels einer zweiten Antriebseinheit 14 entlang der Drehachse 13 translatorisch antreibbar. Hierdurch wird ein zweiter Bewegungsfreiheitsgrad F₂ bereitgestellt. Die Antriebseinrichtungen 12, 14 sind beispielsweise elektrisch ausgebildet.

Die Drehachse 13 unterteilt den Strahlungsquellen-Detektor-Abstand A in einen ersten Abstand A₁ und einen zweiten Abstand A₂. Der erste Abstand A₁ ergibt sich als Abstand der Strahlungsquelle 4 von der Drehachse 13. Entsprechend ergibt sich der zweite Abstand A₂ als Abstand der Drehachse 13 von dem Flächendetektor 6. Der Bauteilträger 7 ist zum Erzeugen einer Projektion S mit einer gewünschten Vergrößerung V entlang der Längsachse 9 zwischen der Strahlungsquelle 4 und dem Flächendetektor 6 verlagerbar. Die Vergrößerung V ergibt sich aus dem Verhältnis von Strahlungsquellen-Detektor-Abstand A zu dem ersten Abstand A₁.

Der Röntgencomputertomograph 1 ist derart ausgebildet, dass der Bauteilträger 7 entlang einer helixförmigen Trajektorie verlagerbar ist. Das bedeutet, dass ein Brennfleck B der Strahlungsquelle 4 relativ zu dem Bauteilträger 7 bzw. dem auf dem Bauteilträger 7 angeordneten Bauteil 2 eine helixförmige Bahn beschreiben kann. Der Bauteilträger 7 ist derart entlang der helixförmigen Trajektorie verlagerbar, dass ein Verhältnis P von Vorschub Δy pro Umdrehung entlang der Drehachse 13 zu Strahlaufweitung Y der auf dem Flächendetektor 6 detektierbaren Röntgenstrahlung 5 entlang der Drehachse 13 kleiner als 1,5, insbesondere kleiner als 1, insbesondere kleiner als 0,85, und insbesondere kleiner als 0,7, ist. Dieses Verhältnis wird auch als Pitch P bzw. relativer Pitch P bezeichnet. Der Pitch P stellt somit ein Maß für die Steilheit der helixförmigen Trajektorie dar. Ein Pitch P von 1 bedeutet beispielsweise, dass der Bauteilträger 7 pro Umdrehung um die Strahlaufweitung Y verlagert wird. Die Strahlaufweitung Y kann maximal so groß wie das Verhältnis von Detektorhöhe H zu Vergrößerung V sein.

Die Strahlungsquellen-Detektor-Einheit 3 und die Antriebseinrichtungen 12, 14 sind über Signalleitungen 15 mit einer Recheneinrichtung 16 verbunden. Die Recheneinrichtung 16 dient zum Steuern des Röntgencomputertomographen 1 und zum Auswerten der detektierten Röntgenstrahlung 5.

Die Recheneinrichtung 16 umfasst eine Rekonstruktionseinheit 17 und eine Geometrieerfassungseinheit 18. Die Rekonstruktionseinheit 17 ist derart ausgebildet, dass aus der entlang der helixförmigen Trajektorie detektierten Röntgenstrahlung 5 ein dreidimensionales Röntgenbild des zu untersuchenden Bauteils 2 rekonstruierbar ist. Hierzu ist in der Rekonstruktionseinheit 17 ein Helix-Rekonstruktionsalgorithmus implementiert. Ein derartiger Rekonstruktionsalgorithmus ist prinzipiell bekannt und beispielsweise in der Dissertation "Performance Evaluation of Exact and Approximate Cone-Beam Algorithmus in Spiral Computed Tomography" von Katia Sourbelle, Shaker Verlag, Aachen, 2002, beschrieben.

Die Geometrieerfassungseinheit 18 dient zum Bestimmen von dimensionellen Messdaten M des zu untersuchenden Bauteils 2 aus dem rekonstruierten dreidimensionalen Röntgenbild. Die Geometrieerfassungseinheit 18 ist derart ausgebildet, dass eine Bauteiloberfläche des Bauteils 2 ermittelbar ist. Die Bauteiloberfläche ist insbesondere aus verbundenen Dreiecksflächen ermittelbar.

Die Verdrehstellung des Bauteilträgers 7 und somit des Bauteils 2 wird durch einen Verdrehwinkel ϕ gekennzeichnet. Der Verdrehwinkel ϕ ist ein Maß für die jeweilige Projektionsrichtung.

Nachfolgend wird die geometrische Vermessung des Bauteils 2 mittels des erfindungsgemäßen Röntgencomputertomographen 1 beschrieben.

Das zu untersuchende Bauteil 2 wird auf dem Bauteilträger 7 angeordnet. Das Bauteil 2 ist beispielweise aus Kunststoff oder Metall. In Abhängigkeit der Größe des Bauteils 2 und der gewünschten Vergrößerung V der Projektion S wird der Strahlungsquellen-Detektor-Abstand A sowie der Kegelöffnungswinkel α eingestellt und der Bauteilträger 7 in einer gewünschten Position in der z-Richtung positioniert.

Zum Erzeugen einer helixförmigen Trajektorie wird mittels der Recheneinrichtung 16 ein Pitch P eingestellt, entsprechend dem eine helixförmige

Trajektorie mit einer gewünschten Steilheit erzeugbar ist. Entsprechend dem Pitch P wird der Flächendetektor 6 um die Schwenkachse 11 verschwenkt, so dass der Flächendetektor 6 tangential zu der helixförmigen Trajektorie ausgerichtet ist. Nach dem Ausrichten weisen die Zeilen des Flächendetektors 6 somit eine der Steilheit der helixförmigen Trajektorie entsprechende Steilheit auf.

Mittels der Strahlungsquelle 4 wird das Bauteil 2 mit Röntgenstrahlung 5 bestrahlt, wobei die Röntgenstrahlung 5 kegelförmig mit dem gewünschten Kegelöffnungswinkel α austritt. Das Bestrahlen erfolgt in Abhängigkeit des Materials und der Größe des Bauteils 2 mit einer Beschleunigungsspannung U_{B} von mehr als 150 kV, insbesondere von mehr als 450 kV, und insbesondere von mehr als 900 kV.

Während des Bestrahlens steuert die Recheneinrichtung 16 die Antriebseinrichtungen 12, 14 so an, dass der Bauteilträger 7 gleichzeitig um die Drehachse 13 gedreht und entlang der Drehachse 13 in y-Richtung translatorisch bewegt wird. Das Bauteil 2 wird somit relativ zu dem Brennfleck B der Strahlungsquelle 4 entlang einer helixförmigen Trajektorie bewegt, wobei die helixförmige Trajektorie eine Steilheit entsprechend dem eingestellten Pitch P aufweist. Die Anzahl der erforderlichen Umdrehung um die Drehachse 13 ergibt sich aus der Größe des Bauteils 2 in y-Richtung, der Strahlaufweitung Y und dem Pitch P.

Die Röntgenstrahlung 5 wird mittels des Flächendetektors 6 detektiert und in digitale Volumendaten gewandelt. Die Volumendaten werden der Recheneinrichtung 16 zugeführt. Mittels der Rekonstruktionseinheit 17 wird aus den Volumendaten ein dreidimensionales Röntgenbild des Bauteils 2 rekonstruiert. Hierzu werden die Volumendaten zunächst in tangentialer Richtung zu der helixförmigen Trajektorie gefiltert. Durch den ausgerichteten Flächendetektor 6 kann diese Filterung entlang der Pixel 10 einer Zeile durchgeführt werden, so dass ein zusätzlicher Interpolationsschritt zwischen den Pixeln 10, wie dies bei einem nicht ausgerichteten Flächendetektor 6 erforderlich wäre, entfällt. Die erzielbare Auflösung wird durch die Filterung somit nicht beeinträchtigt. Die gefilterten Volumendaten werden dem Helix-Rekonstruktionsalgorithmus zugeführt und zu einem dreidimensionalen Röntgenbild umgerechnet.

Die Rekonstruktion des dreidimensionalen Röntgenbildes aus den Volumendaten ist prinzipiell in Fig. 3 gezeigt. In Fig. 3 sind beispielhaft Strahlenbereiche Δα dargestellt, in denen jeweils Röntgenstrahlen verlaufen, die im Bereich eines Pixels 10 auf den Flächendetektor 6 treffen und für die in Fig. 3 gezeigte Projektionsstellung entsprechende Volumendaten erzeugen. Bei der Rekonstruktion wird jede Volumeneinheit (Voxel) des dreidimensionalen Röntgenbildes unmittelbar und ausschließlich aus Volumendaten zu solchen Röntgenstrahlen berechnet, die an der der Volumeneinheit entsprechenden Stelle durch das Bauteil 2 bzw. die Bauteilumgebung verliefen. Anders ausgedrückt, durchläuft beispielsweise eine bestimmte - in Fig. 3 mit ΔV bezeichnete - Stelle des Bauteils 2 während ihrer helixförmigen Relativbewegung T zu dem Brennfleck B mehrere Strahlenbereiche Δα, in denen die Röntgenstrahlen durch diese Stelle im Bauteil 2 verlaufen und entsprechende Volumendaten erzeugen. Bei der Rekonstruktion der dieser Stelle entsprechenden Volumeneinheit werden diese Volumendaten unmittelbar in die Volumeneinheit umgerechnet. Zwischenschritte, wie beispielsweise das Rekonstruieren und Zusammenfügen von Schichten des Bauteils 2, sind nicht erforderlich, sodass die Rekonstruktion des Röntgenbildes schnell und mit einer hohen Genauigkeit möglich ist.

Bei der Auswertung des Röntgenbildes können bereits fehlerhafte innere Strukturen des Bauteils 2 lokalisiert und charakterisiert werden.

Mittels der Geometrieerfassungseinheit 18 werden aus dem rekonstruierten Röntgenbild dimensionale Messdaten M von inneren Strukturen des Bauteils 2, wie beispielsweise Längen, Flächen oder unerwünschte Abweichungen von einer gewünschten Form, bestimmt. Die Geometrieerfassungseinheit 18 ermittelt zunächst die Bauteiloberfläche des Bauteils 2. Die Bauteiloberfläche setzt sich aus der äußeren und der inneren - nicht zugänglichen - Bauteiloberfläche zusammen. Hierzu wird jeder Volumeneinheit (Voxel) des dreidimensionalen Röntgenbildes ein Dichtewert zugeordnet. Anhand der Dichtewerte ist das Material des Bauteils 2 von der umgebenden Luft unterscheidbar, so dass sich die Bauteiloberfläche als Fläche gleicher Dichtewerte ergibt. Die Bauteiloberfläche wird anschließend durch miteinander verbundene Dreiecke angenähert, wodurch der Speicher- und Rechenaufwand zum geometrischen Vermessen der Bauteiloberfläche optimiert wird. Das Bestimmen der Dreiecksflächen erfolgt derart, dass alle Volumeneinheiten des dreidimensionalen Röntgenbildes durchlaufen werden, wobei für die Volumeneinheiten, die von der Fläche gleicher Dichtewerte geschnitten werden, die darin enthaltene Teilfläche durch Dreiecksflächen approximiert wird. Aus der Bauteiloberfläche können anschließend die dimensionalen Messdaten M gewonnen werden.

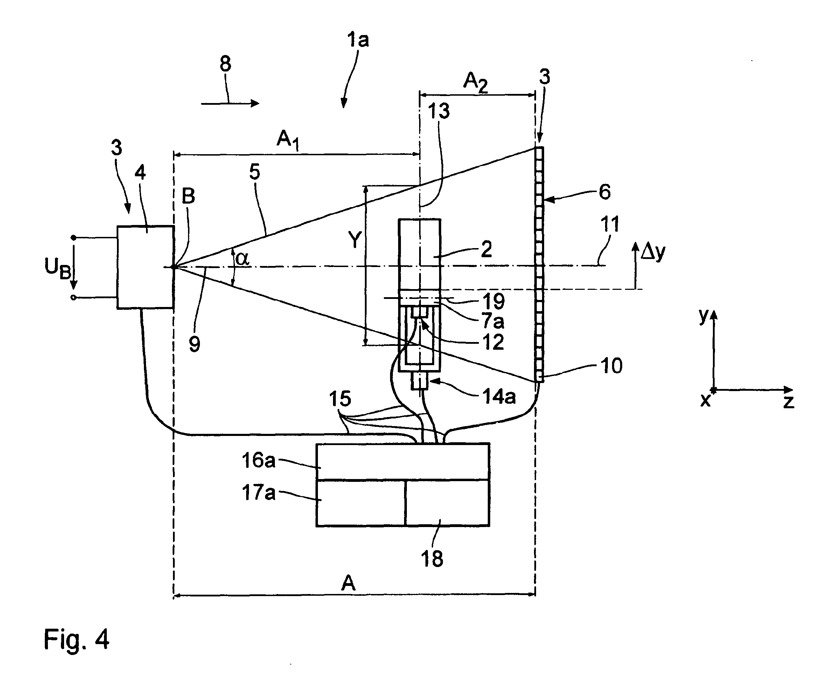

Nachfolgend wird unter Bezugnahme auf Fig. 4 ein zweites Ausführungsbeispiel der Erfindung beschrieben. Konstruktiv identische Teile erhalten dieselben Bezugszeichen wie bei dem ersten Ausführungsbeispiel, auf dessen Beschreibung hiermit verwiesen wird. Konstruktiv unterschiedliche, jedoch funktionell gleichartige Teile erhalten dieselben Bezugszeichen mit einem nachgestellten a. Der wesentliche Unterschied gegenüber dem ersten Ausführungsbeispiel besteht darin, dass als zweiter Bewegungsfreiheitsgrad F₂ der Bauteilträger 7a des Röntgencomputertomographen 1a um eine parallel zu der Längsachse 9 verlaufende Schwenkachse 19 mittels der Antriebseinrichtung 14a verschwenkbar ist. Zusätzlich kann der Bauteilträger 7a als dritter Bewegungsfreiheitsgrad F₃ - entsprechend dem ersten Ausführungsbeispiel - entlang der Drehachse 13 verlagerbar sein. Die Schwenkachse 19 verläuft senkrecht zu der Drehachse 13, so dass mehrere kreisförmige und zueinander geneigte Trajektorien erzeugbar sind. Die Rekonstuktionseinheit 17a der Recheneinrichtung 16a ist derart ausgebildet, dass aus der entlang zweier kreisförmiger und zueinander geneigter Trajektorien detektierten Röntgenstrahlung 5 ein dreidimensionales Röntgenbild rekonstruierbar ist.

Zum Bestrahlen des Bauteils 2 befindet sich der Bauteilträger 7a zunächst in der in Figur 4 gezeigten Position. In dieser Position wird der Bauteilträger 7a mittels der Antriebseinrichtung 12 einmal um die Drehachse 13 gedreht, so dass das Bauteil 2 relativ zu dem Brennfleck B entlang einer ersten kreisförmigen Trajektorie bewegt wird. Die derart gewonnenen Volumendaten werden der Recheneinrichtung 16a zugeführt. Anschließend wird der Bauteilträger 7a mittels der Antriebseinrichtung 14a um 90 ° um die Schwenkachse 19 verschwenkt. In dieser Position wird das Bauteil 2 erneut bestrahlt und einmal um die um 90 ° geschwenkte Drehachse 13 gedreht. Der Flächendetektor 6 kann entsprechend dem Bauteilträger 7a um die Schwenkachse 11 verschwenkt werden, so dass die beim erneuten Bestrahlen gewonnenen Volumendaten einfach mittels der Rekonstruktionseinheit 17a zu einem dreidimensionalen Röntgenbild rekonstruierbar sind. Die beim erneuten Bestrahlen gewonnenen Volumendaten werden der Recheneinrichtung 16a zugeführt. Bei dem erneuten Bestrahlen des Bauteils 2 wird dieses relativ zu dem Brennfleck B entlang einer zweiten kreisförmigen Trajektorie bewegt, wobei die zweite Trajektorie relativ zu der ersten Trajektorie um 90 ° geneigt ist. Aus den Volumendaten rekonstruiert die Rekonstruktionseinheit 17a ein dreidimensionales Röntgenbild, das entsprechend zu dem ersten Ausführungsbeispiel ein geometrisches Vermessen des Bauteils 2 mittels der Geometrieerfassungseinheit 18 ermöglicht.

Hinsichtlich der weiteren Funktionsweise, insbesondere der Rekonstruktion des dreidimensionalen Röntgenbildes, wird auf das erste Ausführungsbeispiel verwiesen.

Die Erfindung ermöglicht somit eine enorme Geschwindigkeitssteigerung im Vergleich zum geometrischen Vermessen des Bauteils 2 mittels eines zweidimensionalen Fächerstrahls in Verbindung mit einer hohen Messgenauigkeit. Insbesondere wird im Vergleich zur Untersuchung des Bauteils 2 mittels eines dreidimensionalen Kegelstrahls bei ausschließlich einer Rotation des Bauteils 2, also bei einer einen nur zweidimensionalen Raum aufspannenden Trajektorie, der Anteil an Kegelstrahlartefakten, die bei feldkampartigen Rekonstruktionsalgorithmen unvermeidbar sind, deutlich reduziert und die geometrische Genauigkeit der Rekonstruktion, insbesondere außerhalb der zentralen Strahlungsebene, deutlich verbessert. Die geometrische Auflösung ist für das gesamte Bauteil 2 homogen und isotrop. Die Erfindung ermöglicht somit den Einsatz der dimensionellen Messtechnik (Metrologie) in industriellen Anwendungen, da dimensionelle Messdaten schnell und ortsunabhängig mit hoher Genauigkeit bestimmt werden können.

## Patentansprüche

1. Röntgencomputertomograph zur Untersuchung eines Bauteils mittels Röntgencomputertomographie mit
- einer Strahlungsquellen-Detektor-Einheit (3), umfassend
- - eine Strahlungsquelle (4) zum Erzeugen von Röntgenstrahlung (5) mit einer dreidimensionalen Strahlungsgeometrie, und
- einem Flächendetektor (6) zum Detektieren der Röntgenstrahlung (5),
- einem Bauteilträger (7; 7a) zum Positionieren eines zu untersuchenden Bauteils (2) zwischen der Strahlungsquelle (4) und dem Flächendetektor (6), und
- einer Recheneinrichtung (16; 16a) zum Auswerten der detektierten
Röntgenstrahlung (5),
wobei
- die Strahlungsquellen-Detektor-Einheit (3) und der Bauteilträger (7; 7a) relativ zueinander mindestens zwei Bewegungsfreiheitsgrade (F) derart aufweisen, dass mindestens eine einen dreidimensionalen Raum aufspannende Trajektorie erzeugbar ist,
- die Recheneinrichtung (16; 16a)
- - eine Rekonstruktionseinheit (17; 17a) zum Rekonstruieren eines dreidimensionalen Röntgenbildes des Bauteils (2) aus der entlang der mindestens einen Trajektorie detektierten Röntgenstrahlung (5) aufweist, und
- - eine Geometrieerfassungseinheit (18) zum Bestimmen von dimensionellen Messdaten (M) des Bauteils (2) aus dem dreidimensionalen Röntgenbild aufweist,
**dadurch gekennzeichnet, dass** zur geometrischen Vermessung des Bauteils (2)
- der Bauteilträger (7; 7a) entlang einer Längsachse (9) zwischen der Strahlungsquelle (4) und dem Flächendetektor (6) verlagerbar ist und
- der Flächendetektor (6) um eine quer zu diesem verlaufende Schwenkachse (11) verschwenkbar ist.

2. Röntgencomputertomograph nach Anspruch 1, **dadurch gekennzeichnet, dass** die Geometrieerfassungseinheit (18) derart ausgebildet ist, dass eine Bauteiloberfläche des Bauteils (2) ermittelbar ist.

3. Röntgencomputertomograph nach Anspruch 2, **dadurch gekennzeichnet, dass** die Geometrieerfässungseinheit (18) derart ausgebildet ist, dass die Bauteiloberfläche aus verbundenen Dreiecksflächen ermittelbar ist.

4. Röntgencomputertomograph nach einem der Ansprüche 1 bis 3, **dadurch gekennzeichnet, dass** als ein Bewegungsfreiheitsgrad (F) der Bauteilträger (7; 7a) um eine Drehachse (13) drehantreibbar ist.

5. Röntgencomputertomograph nach Anspruch 4, **dadurch gekennzeichnet dass** als ein weiterer Bewegungsfreiheitsgrad (F) der Bauteilträger (7; 7a) entlang der Drehachse (13) translatorisch antreibbar ist.

6. Röntgencomputertomograph nach Anspruch 5, **dadurch gekennzeichnet, dass** der Bauteilträger (7; 7a) derart entlang einer helixförmigen Trajektorie verlagerbar ist, dass ein Verhältnis (P) von Vorschub (Δy) pro Umdrehung zu Strahlaufweitung (Y) entlang der Drehachse (13) kleiner als 1,5, insbesondere kleiner als 1, insbesondere kleiner als 0,85, und insbesondere kleiner als 0,7, ist.

7. Röntgencomputertomograph nach Anspruch 6, **dadurch gekennzeichnet, dass** die Rekonstruktionseinheit (17; 17a) derart ausgebildet ist, dass aus der entlang einer helixförmigen Trajektorie detektierten Röntgenstrahlung (5) das Röntgenbild rekonstruierbar ist.

8. Röntgencomputertomograph nach einem der Ansprüche 4 bis 7, **dadurch gekennzeichnet, dass** als ein weiterer Bewegungsfreiheitsgrad (F) der Bauteilträger (7a) um eine quer zu der Drehachse (13) verlaufende Schwenkachse (19) verschwenkbar ist.

9. Röntgencomputertomograph nach Anspruch 8, **dadurch gekennzeichnet, dass** die Rekonstruktionseinheit (17a) derart ausgebildet ist, dass aus der entlang zweier kreisförmiger und zueinander geneigter Trajektorien detektierten Röntgenstrahlung (5) das Röntgenbild rekonstruierbar ist.

10. Röntgencompütertomograph nach einem der Ansprüche 1 bis 9, **dadurch gekennzeichnet, dass** die Strahlungsquellen-Detektor-Einheit (3) derart ausgebildet ist, dass ein Strahlungsquellen-Detektor-Abstand (A) veränderbar ist.

11. Röntgencomputertomograph nach einem der Ansprüche 1 bis 10, **dadurch gekennzeichnet, dass** die Strahlungsquelle (4) einen Kegelöffnungswinkel (α) von mehr als 10°, insbesondere von mehr als 30°, und insbesondere von mehr als 50°, aufweist.

12. Röntgencomputertomograph nach einem der Ansprüche 1 bis 11, **dadurch gekennzeichnet, dass** die Strahlungsquelle (4) eine Beschleunigungsspannung (U_{B}) von mindestens 150 kV, insbesondere von mindestens 450 kV, und insbesondere von mindestens 900 kV, aufweist.

13. Röntgencomputertomograph nach einem der Ansprüche 1 bis 12, **dadurch gekennzeichnet, dass** die Rekonstruktionseinheit (17; 17a) derart ausgebildet ist, dass jede Volumeneinheit des Röntgenbildes unmittelbar aus mittels des Flächendetektors (6) aufgenommenen Volumendaten zu solchen Röntgenstrahlen rekonstruiert wird, die an der der Volumeneinheit entsprechenden Stelle verliefen.

14. Verfahren zur Untersuchung eines Bauteils mittels Röntgencomputertomographie, umfassend die Schritte:
- Positionieren eines zu untersuchenden Bauteils (2) auf einem Bauteilträger (7; 7a) zwischen einer Strahlungsquelle (4) und einem Flächendetektor (6) einer Strahlungsquellen-Detektor-Einheit (3),
- Verlagern des Bauteilträgers (7; 7a) entlang einer Längsachse (9) zwischen der Strahlungsquelle (4) und dem Flächendetektor (6) und Verschwenken des Flächendetektors (6) um eine quer zu diesem verlaufende Schwenkachse (11) derart, dass das Bauteil (2) geometrisch vermessbar ist,
- Bestrahlen des Bauteils (2) mit Röntgenstrahlung (5) mittels der Strahlungsquelle (4), wobei die Röntgenstrahlung (5) eine dreidimensionale Strahlungsgeometrie aufweist,
- Detektieren der Röntgenstrahlung (5) mittels des Flächendetektors (6), und
- Auswerten der detektierten Röntgenstrahlung (5) mittels einer Recheneinrichtung (16; 16a),
wobei
- die Strahlungsquellen-Detektor-Einheit (3) und das Bauteil (2) relativ zueinander eine Bewegung in mindestens zwei Bewegungsfreiheitsgraden (F) derart ausführen, dass mindestens eine einen dreidimensionalen Raum aufspannende Trajektorie erzeugt wird,
- aus der entlang der mindestens einen Trajektorie detektierten Röntgenstrahlung (5) ein dreidimensionales Röntgenbild des Bauteils (2) mittels einer Rekonstruktionseinheit (17; 17a) der Recheneinrichtung (16; 16a) rekonstruiert wird, und
- aus dem dreidimensionalen Röntgenbild mittels einer Geometrieerfassungseinheit (18) der Recheneinrichtung (16; 16a) dimensionelle Messdaten (M) des Bauteils (2) bestimmt werden.

## Claims

1. X-ray computer tomograph for examining a component by means of X-ray computer tomography, comprising
- a radiation source detector unit (3), comprising
- - a radiation source (4) for producing X-radiation (5) with a three-dimensional radiation geometry, and
- - a planar detector (6) for detecting the X-radiation (5),
- a component carrier (7; 7a) for positioning a component (2) to be examined between the radiation source (4) and the planar detector (6), and
- a computing device (16; 16a) for evaluating the detected X-radiation (5),
wherein
- the radiation source detector unit (3) and the component carrier (7; 7a) have at least two degrees (F) of freedom of movement relative to one another in such a way that at least one trajectory spanning a three-dimensional space is producible,
- the computing device (16; 16a)
- - has a reconstruction unit (17; 17a) for reconstructing a three-dimensional X-ray image of the component (2) from the X-radiation (5) detected along the at least one trajectory, and
- - has a geometry detection unit (18) for determining dimensional measurement data (M) of the component (2) from the three-dimensional X-ray image,
**characterized in that** for the geometric measurement of the component (2)
- the component carrier (7; 7a) is displaceable along a longitudinal axis (9) between the radiation source (4) and the planar detector (6)
and
- the planar detector (6) is pivotable about a pivot axis (11) running transverse thereto.

2. X-ray computer tomograph according to claim 1, **characterised in that** the geometry detection unit (18) is configured in such a way that a component surface of the component (2) is determinable.

3. X-ray computer tomograph according to claim 2, **characterised in that** the geometry detection unit (18) is configured in such a way that the component surface is determinable from connected triangular areas.

4. X-ray computer tomograph according to any one of claims 1 to 3, **characterised in that** the component carrier (7; 7a) is rotatably drivable about a rotational axis (13) as one degree (F) of freedom of movement.

5. X-ray computer tomograph according to claim 4, **characterised in that** the component carrier (7; 7a) is drivable in a translatory manner along the rotational axis (13) as a further degree (F) of freedom of movement.

6. X-ray computer tomograph according to claim 5, **characterised in that** the component carrier (7; 7a) is displaceable along a helical trajectory in such a way that a ratio (P) of feed (Δy) per rotation to beam widening (Y) along the rotational axis (13) is less than 1.5, in particular less than 1, in particular less than 0.85, and in particular less than 0.7.

7. X-ray computer tomograph according to claim 6, **characterised in that** the reconstruction unit (17; 17a) is configured in such a way that the X-ray image is reconstructable from the X-radiation (5) detected along a helical trajectory.

8. X-ray computer tomograph according to any one of claims 4 to 7, **characterised in that** the component carrier (7a) is pivotable about a pivot axis (19) running transverse to the rotational axis (13) as a further degree (F) of freedom of movement.

9. X-ray computer tomograph according to claim 8, **characterised in that** the reconstruction unit (17a) is configured in such a way that the X-ray image is reconstructable from the X-radiation (5) detected along two circular trajectories inclined with respect to one another.

10. X-ray computer tomograph according to any one of claims 1 to 9, **characterised in that** the radiation source detector unit (3) is configured in such a way that a radiation source detector spacing (A) is changeable.

11. X-ray computer tomograph according to any one of claims 1 to 10, **characterised in that** the radiation source (4) has a cone opening angle (α) of more than 10°, in particular of more than 30°, and in particular of more than 50°.

12. X-ray computer tomograph according to any one of claims 1 to 11, **characterised in that** the radiation source (4) has an accelerating voltage (U_{B}) of at least 150 kV, in particular of at least 450 kV, and in particular of at least 900 kV.

13. X-ray computer tomograph according to any one of claims 1 to 12, **characterised in that** the reconstruction unit (17; 17a) is configured in such a way that each volume unit of the X-ray image is reconstructed directly from volume data recorded by means of the planar detector (6) on X-rays of the type which ran at the point corresponding to the volume unit.

14. Method for examining a component by means of X-ray computer tomography, comprising the steps:
- positioning a component (2) to be examined on component carrier (7; 7a) between a radiation source (4) and a planar detector (6) of a radiation source detector unit (3),
- displacing the component carrier (7; 7a) along a longitudinal axis (9) between the radiation source (4) and the planar detector (6) and pivoting the planar detector (6) about a perpendicular pivot axis (11) in such a way, that the component (2) is geometrically measurable,
- irradiating the component (2) with X-radiation (5) by means of the radiation source (4), the X-radiation (5) having a three-dimensional radiation geometry,
- detecting the X-radiation (5) by means of the planar detector (6),
and
- evaluating the detected X-radiation (5) by means of a computing
device (16; 16a)
wherein
- the radiation source detector unit (3) and the component (2) carry out a movement relative to one another in at least two degrees (F) of freedom of movement, in that at least one trajectory spanning a three-dimensional space is produced,
- a three-dimensional X-ray image of the component (2) is reconstructed from the X-radiation (5) detected along the at least one trajectory by means of a reconstruction unit (17; 17a) of the computing device (16; 16a), and
- dimensional measurement data (M) of the component (2) are determined from the three-dimensional X-ray image by means of a geometry detection unit (18) of the computing device (16; 16a).

## Revendications

1. Tomodensitomètre à rayons X pour le contrôle d'un élément structurel au moyen de la tomodensitométrie avec
- un ensemble source de rayonnement-détecteur (3) comprenant
- - une source de rayonnement (4) pour la génération d'un rayonnement X (5) avec une géométrie de rayonnement tridimensionnelle, et
- - un détecteur de surface (6) pour la détection du rayonnement X (5),
- un support d'élément structurel (7 ; 7a) pour le positionnement d'un élément structurel (2) à contrôler entre la source du rayonnement (4) et le détecteur de surface (6), et
- une unité de calcul (16 ; 16a) pour l'exploitation du rayonnement X détecté (5),
où
- l'ensemble source de rayonnement-détecteur (3) et le support d'élément structurel (7 ; 7a) présentent l'un par rapport à l'autre au moins deux degrés de liberté (F) de mouvement, de sorte qu'au moins une trajectoire s'étendant dans un espace tridimensionnel peut être générée,
- l'unité de calcul (16 ; 16a)
- - présente une unité de reconstruction (17 ; 17a) pour la reconstruction d'une image tridimensionnelle par rayons X de l'élément structurel, à partir de laquelle au moins un rayonnement X (5) détecté par la trajectoire, et
- - présente une unité de saisie de la géométrie (18) pour l'évaluation des données de mesure (M) dimensionnelles de l'élément structurel (2) à partir de l'image par rayons X tridimensionnelle
**caractérisé en ce que**, pour la prise des dimensions géométriques de l'élément structurel (2)
- le support d'élément structurel (7 ; 7a) peut être déplacé le long d'un axe longitudinal (9) entre la source du rayonnement (4) et le détecteur de surface (6) et
- le détecteur de surface (6) peut être orientable autour d'un axe orientable (11) qui lui est perpendiculaire.

2. Tomodensitomètre à rayons X selon la revendication 1, **caractérisé en ce que** l'unité de saisie de la géométrie (18) est construite de sorte qu'une surface d'élément structurel de l'élément structurel (2) peut être déterminée.

3. Tomodensitomètre à rayons X selon la revendication 2 **caractérisé en ce que** l'unité de saisie de la géométrie (18) est construite de sorte que la surface d'élément structurel peut être déterminée à partir de surfaces triangulaires associées.

4. Tomodensitomètre à rayons X selon l'une des revendications de 1 à 3, **caractérisé en ce qu'**un degré de liberté de mouvement (F) du support d'élément structurel (7 ; 7a) peut être actionné par rotation autour d'un axe de rotation (13).

5. Tomodensitomètre à rayons X selon la revendication 4 **caractérisé en ce qu'**un autre degré de liberté de mouvement (F) du support d'élément structurel (7 ; 7a) peut être déplacé de manière translatoire le long de l'axe de rotation (13).

6. Tomodensitomètre à rayons X selon la revendication 5 **caractérisé en ce que** le support d'élément structurel (7 ; 7a) peut être déplacé le long d'une trajectoire en forme d'hélice, de sorte qu'un rapport (P) entre l'avance (Δy) par tour de rotation et l'élargissement du rayonnement (Y) le long de l'axe de rotation (13) est inférieur à 1,5, en particulier inférieur à 1, en particulier inférieur à 0,85, et en particulier inférieur à 0,7.

7. Tomodensitomètre à rayons X selon la revendication 6 **caractérisé en ce que** l'unité de reconstruction (17 ; 17a) est construite de sorte que l'image par les rayons X peut être reconstruite à partir du rayonnement X (5) détecté le long d'une trajectoire en hélice.

8. Tomodensitomètre à rayons X selon l'une des revendications de 4 à 7 **caractérisé en ce qu'**un autre degré de liberté de mouvement (F) du support d'élément structurel (7a) peut être orientable autour d'un axe orientable (19) perpendiculaire à l'axe de rotation (13).

9. Tomodensitomètre à rayons X selon la revendication 8 **caractérisé en ce que** l'unité de reconstruction (17a) est construite de sorte que l'image par rayons X peut être reconstruite à partir du rayonnement X (5) détecté le long de deux trajectoires circulaires et inclinées l'une vers l'autre.

10. Tomodensitomètre à rayons X selon l'une des revendications de 1 à 9 **caractérisé en ce que** l'ensemble source de rayonnement-détecteur (3) est conçu de sorte qu'une distance (A) source de rayonnement -détecteur peut être variée.

11. Tomodensitomètre à rayons X selon l'une des revendications de 1 à 10 **caractérisé en ce que** la source de rayonnement (4) présente un angle d'ouverture conique (α) de plus de 10 °, en particulier de plus de 30 °, et particulièrement de plus de 50 °.

12. Tomodensitomètre à rayons X selon l'une des revendications de 1 à 11 **caractérisé en ce que** la source de rayonnement (4) présente une tension d'accélération (U_{B}) d'au minimum 150 kV, en particulier d'au minimum 450 kV, et particulièrement d'au moins 900 kV.

13. Tomodensitomètre à rayons X selon l'une des revendications de 1 à 12 **caractérisé en ce que** l'unité de reconstruction (17 ; 17a) est construite de sorte que chaque unité de volume de l'image par rayons X est reconstruite directement à partir de données volumiques enregistrées grâce au détecteur de surface (6) en des rayonnements X tels que ceux qui ont passé au point correspondant dans l'unité de volume.

14. Procédé de contrôle d'un élément structurel par tomodensitométrie à rayons X comprenant les étapes :
- de positionnement d'un élément structurel (2) à contrôler sur un support d'élément structurel (7 ; 7a) entre une source de rayonnement (4) et un détecteur de surface (6) d'un ensemble source de rayonnement-détecteur (3),
- de déplacement du support d'élément structurel (7, 7a) le long d'un axe longitudinal (9) entre une source de rayonnement (4) et le détecteur de surface (6) et orientation du détecteur de surface (6) autour d'un axe orientable (11) se situant perpendiculairement à lui, de sorte que l'élément structurel (2) peut être mesuré géométriquement,
- de l'irradiation de l'élément structurel (2) par un rayonnement X (5) grâce à une source de rayonnement (4), le rayonnement X (5) présentant une géométrie de rayonnement tridimensionnelle,
- de la détection du rayonnement X (5) grâce à un détecteur de surface (6), et
- de l'exploitation du rayonnement X (5) détecté au moyen d'une unité de calcul (16 ; 16a),
où
- l'ensemble source de rayonnement- détecteur (3) et l'élément structurel (2), relativement l'un par rapport à l'autre, réalisent un déplacement dans au moins deux degrés de liberté de mouvement (F) de telle manière qu'au minimum une trajectoire s'étendant dans un espace tridimensionnel est générée,
- une image par rayons X tridimensionnelle de l'élément structurel (2) est reconstruite à partir du rayonnement X (5) détecté le long d'au moins une trajectoire, au moyen d'une unité de reconstruction (17 ; 17a) de l'unité de calcul (16 ; 16a), et
- des données de mesures dimensionnelles (M) de l'élément structurel (2) sont évaluées à partir de l'image par rayons X tridimensionnelle grâce à une unité de saisie géométrique (18) de l'unité de calcul (16 ; 16a).
